# EUROPEAN PATENT SPECIFICATION

(11) **EP 1 802 364 B1**
(45) Date of publication and mention of the grant of the patent: **15.09.2010**
(21) Application number: 04784846.0
(22) Date of filing: 22.09.2004
(51) Int. Cl.: A61M 5/00

(54) **SAFETY ADAPTER FOR NEEDLE HUB ASSEMBLY**
SICHERHEITSADAPTER FÜR EINE NADELANSATZ-ANORDNUNG
ADAPTATEUR DE SURETE POUR ENSEMBLE PORTE-AIGUILLE

(43) Date of publication of application: 04.07.2007
(73) Proprietor: Smiths Medical ASD, Inc., Keene, NH 03431-0724 (US)
(72) Inventor: HUDON, Lawrence, P, Hinsdale, NH 03451 (US)
(74) Representative: Peel, James Peter
(86) International application number: PCT/US2004/031158
(87) International publication number: WO 2006/041442

(56) References cited:
- US-A- 5 405 332
- US-A- 5 632 732
- US-A- 5 681 295

## Description

### Field of the Invention

The present invention relates to a needle protection device and more particularly to an adapter that is fitted to a conventional needle hub assembly so that the assembly may be retrofitted with or added thereto a needle protection device when it is used with a syringe.

### Background of the Invention

To prevent a contaminated needle from being exposed, needle protection housings are used. U.S. patent 4,982,842 discloses an adapter to which a needle hub assembly and a syringe are mated. A needle protection housing is hingedly connected to the adapter so that the housing may be pivoted to cover the needle extending from the needle hub once it has been used. The needle housing for the adapter of the '842 patent is not rotatable. U.S. patent 5,277,311 discloses a housing attached to a collar, with the collar mounted about the neck of a Vacutainer holder, so that the housing is rotatable with respect to a double ended needle mated to the Vacutainer holder. U.S. patent 5,681,295 discloses a needle hub especially made to include a collar receiving recess so that a collar may be fitted directly about the needle hub. A needle protection housing is connected to the collar.

### Summary of the Present Invention

The present invention is defined in claim 1 and allows the retrofitting of a needle protection housing to a conventional needle hub assembly, without the disadvantages of the prior art which requires, among other things, the design of a special needle hub. To achieve this end, the safety device of the instant invention has a sleeve that is adaptable to fit over a conventional needle hub. The sleeve has a proximal portion that is dimensioned to fit to the hub of a conventional needle assembly. The sleeve also has a distal portion that has an opening dimensioned to allow the needle cap or sheath of a conventional needle assembly to pass through so that the base of the cap would frictionally couple to the hub of the needle assembly, thereby covering the needle before its use. A groove or recess is formed at the outer surface of the distal portion of the sleeve to allow a collar, or ring to rotatably mount thereabout. Attached to the collar, byway of a living hinge, is a needle protection sheath or housing, which may have at least one integral hook formed therein.

To use a needle assembly outfitted with the above disclosed sleeved housing, the cap protecting the needle is removed, and the needle protection housing is rotated to an appropriate orientation so that the user can view clearly the tip of the needle for insertion to the patient. After use and after the withdrawal of the needle from the patient, the user only needs to pivot the needle protection housing into an alignment position with respect to the longitudinal axis of the sleeve, or the needle hub assembly, so that the needle protection housing covers the needle. Once covered by the housing and held by the internal hook of the housing, the needle is no longer removable from the needle protection housing. The syringe could then be discarded.

In an alternative embodiment of the invention, instead of an internal hook in the needle protection housing , openings or apertures may be provided at the lower portion of the housing that matingly latch with locking members formed at either the distal portion of the sleeve, or the collar of the housing, so that when the needle protection housing is pivoted to cover the needle, the openings at the base of the housing will coact with the locking member at the sleeve or the collar to fixedly retain the needle protection housing in the alignment position to ensure that the needle is securely covered. The second embodiment may be combined with the first embodiment in that both integral hook(s) and side locking members may be provided to retain the needle protection housing in place to cover the contaminated needle.

### Brief Description of the Figures

Fig 1 is a side view of the sleeve assembly of the instant invention;

Fig. 2 is a side view of the inventive sleeve assembly in relationship to a conventional needle hub assembly;

Fig. 3 is a side view of a needle hub assembly outfitted with the sleeve assembly of the instant invention about to be mated to a conventional syringe shown without its plunger; and

Fig. 4 is a perspective view of a needle hub assembly outfitted with the sleeve assembly of the instant invention mated with a syringe.

### Detailed Description of the Invention

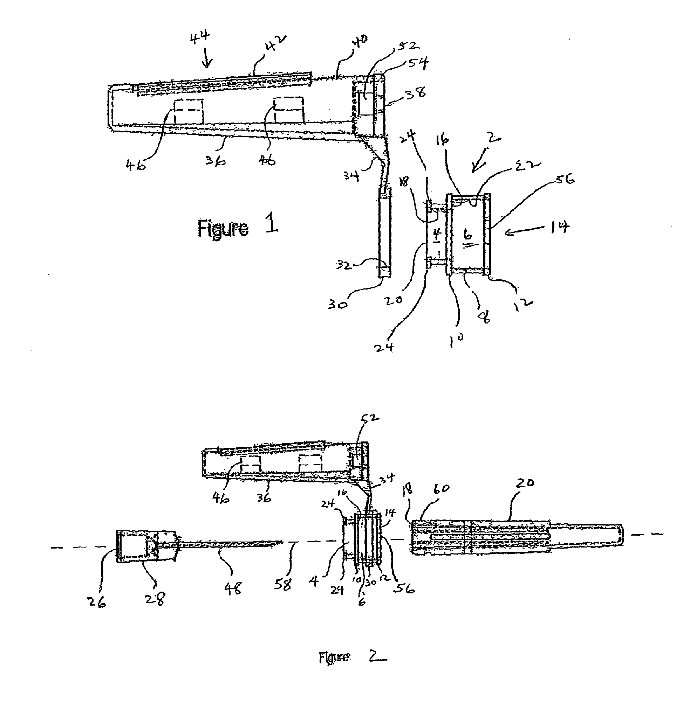

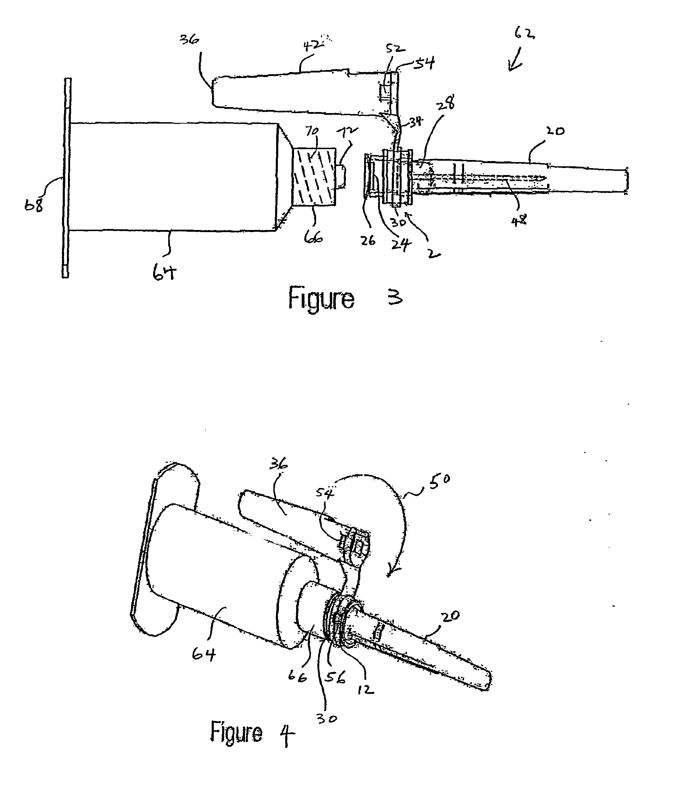

With reference to Fig. 1, the sleeve assembly of the instant invention is shown to include a sleeve 2 having a proximal section 4 and a distal portion 6. The distal portion is shown to have a groove or recess 8 bounded by two raised ends 10 and 12. The sleeve is cylindrical and has a through bore indicated by directional arrow 14. At the intersection of the proximal portion 4 and the distal portion 6 there is an internal circumferential shoulder 16 onto which the base 18 of a needle sheath or cap 20 abuts, when inserted to the opening indicated by directional arrow 14. Proximal section 4 has a first internal diameter 18, indicated by the dotted lines extending from the intersection of the proximal portion 4 and the distal portion 6, and a proximal opening 20. Distal portion 6 has an internal diameter 22 that is larger than diameter 18 of the proximal portion 4. Ears or flanges 24 are provided at the base of proximal portion 4. These flanges are designed to overlay, or superpose, over the flanges or ears 26 of the needle hub 28 of the needle hub assembly shown in Figs. 2 and 3.

A collar or ring 30 is fitted to sleeve 2 about groove 8. Collar 30 has an internal diameter 32 that is slightly wider than the diameter of recess 8 so as to enable it to rotate relative to sleeve 2. Collar 30 is able to be press fit to groove 8 since both collar 30 and sleeve 2 are made of a plastic material such as polypropylene that has certain elastic characteristics that allow the collar to stretch over raised end 10 and then return to its original shape, when collar 30 is mounted about groove 8. Although shown in the drawings to have a width narrower than the width of recess 8, it should be appreciated that collar 30 may actually have a width that allows it to fully seat about groove 8.

Attached to collar 30, by way of a living hinge 34, is a needle protection housing 36. The needle protection housing 36 is exemplified by the housing that is disclosed in the aforementioned '842 patent.
In particular, housing 36 may be made of the same material as collar 30 and is substantially cylindrical in shape. At its bottom 38, housing 36 is open. An elongate slot 40 extends from opening 38 to the distal portion of housing 36. Slot 40 may be covered by a trap door 42, which may open in the direction as indicated by directional arrow 44, but could not be opened in the reverse direction.

Alternatively or in addition, a plurality of needle latching mechanisms such as hooks 46 are integrated to housing 36 for grasping a needle such as needle 48 that extends from needle hub 28, when housing 36 is pivoted in the direction as indicted by directional arrow 50 (Fig. 4) to cover needle 48, assuming that needle cap 20 shown in Fig. 4 has been removed and needle 48 is exposed. There may also be formed apertures or openings 52 at the base 54 of housing 36. Apertures 52 will coact with fingers 56 formed at the distal end of distal portion 6, or on collar 30. Finger 56 and its opposite member on the other side of sleeve 2 would extend into aperture 52 and latch onto base 54, when housing 36 is pivoted along the direction indicated by arrow 50 to be in alignment along the longitudinal axis 58 of sleeve 2 or the needle hub assembly, when housing 36 is moved to cover needle 48.

As discussed above, the opening 14 of distal portion 6 is dimensioned to have a diameter sufficiently large to enable needle cap 20, or more precisely the base portion 60 of the cap, to insert thereinto so that base portion 60 is frictionally coupled to needle hub 28. As shown in Fig. 3, sleeve 2 has been fitted about needle hub 28. Once frictionally coupled to needle 28, since base portion 60 of needle cap 20 has a diameter that is larger than the internal shoulder 16 formed by the intersection of distal portion 6 and proximal portion 4 of sleeve 2, the sleeve assembly is held fixedly by the needle hub 28 coupled with needle cap 20. Thus, the combination sleeve assembly and needle hub assembly as shown per assembled in Fig. 3, after sterilization, can be shipped as one unit.

The unit thus assembled, designated 62, has a sleeve 2 that is rotatable about the needle hub 28. At the same time, collar 30, which is mounted about sleeve 2, is rotatable about sleeve 2, subject to a force applied thereagainst, as the tolerances of the outer circumference of groove 8 and the inner diameter 32 of the collar are such that collar 30 is not freely rotatable about sleeve 2. Housing 36 therefore would remain at a given orientation with respect to needle 48, once the torque forced is removed.

As shown in Fig. 3, the assembled unit of sleeve assembly/needle hub assembly is used with a fluid collecting or dispensing container such as a syringe 64.
Syringe 64 has at one end a luer connector 66, or simply luer, and at the other end an opening 68 for accepting a plunger, not shown. Luer 66, as conventionally known, is internally threaded. The internal groove 70 is configured to accept flange or lip 26 of needle hub 28, so that as needle hub 28 threadedly mates with luer 66, the male portion 72 of luer 66 is inserted into the cavity of needle hub 28 to establish a through passage between needle 48 and syringe 64. As flange 26 is being threaded to luer 66, given that flange 24 of sleeve 2 overlies flange 26, sleeve 2 likewise is threadedly mated to luer 66. As a consequence, before use, a syringe could be fitted with a combination sleeve assembly and needle hub assembly of the instant invention, per shown in Fig. 4.

To use, needle cap 20 is removed. Thereafter, the phlebotomist would insert needle 48 to the patient. After the medicament in syringe 64 has been infused to the patient or blood (or other fluid) withdrawn from the patient, needle 48 is withdrawn and housing 36 is pivoted along the direction indicated by directional arrow 50 to cover the contaminated needle. Depending on the embodiment of housing 36, the needle could snap over the trap door 32 and be trapped within the interior of housing 36. Alternatively or in addition thereto, the needle could be grasped by hooks 46, as it snaps past the tips of hooks 46.

In place of, or in addition to hooks 46, the locking mechanism provided by the combination of apertures 52 and catches 56 enables housing 36 to be retained at the alignment position, once pivoted to that position, to cover the contaminated needle 48. To ensure that each of apertures 54 coacts with a corresponding catch 56, a plurality of catches 56 may be formed about the distal lip 12 of distal portion 6 so that no matter the orientation of housing 36 with respect to the tip of the needle 48, when pivoted to the alignment position per the direction indicated by directional arrow 50, apertures 52 are guaranteed to coact with corresponding catches 56. Once securely covered, the contaminated needle, and the syringe, may be discarded.

By retrofitting a conventional needle hub assembly with the inventive sleeve assembly, an inexpensive needle protection device that allows rotation of a needle protection housing relative to the needle is provided. At the same time, the configuration of the needle hub need not be altered. Further, no medicament is wasted in the case of the syringe being used for dispensing fluid to the patient, as the inventive sleeve does not affect the size or shape of the conventional needle hub. The same is true when a syringe is used to withdraw fluid from the patient in that no withdrawn fluid needs to be wasted. As a consequence, the inventive sleeve assembly is not affected by any dead space issue when dispensing fluid to or withdrawing fluid from a patient.

## Claims

1. A needle protection device, comprising:
a sleeve (2) fittable about a needle hub (28) wherefrom a needle (48) extends, said sleeve having at least one flange (24) that overlies a flange (26) extending from the proximal end of said needle hub; and
a needle protective housing (36) attached to a ring (30) rotatably mounted about said sleeve;
wherein said sleeve is threadedly mated to a luer (66) of a fluid collecting or dispensing container (64) at the same time or after said needle hub has mated to said luer so that said sleeve is fixedly coupled to said container along with said needle hub while said needle protective housing is rotatable about said sleeve, said needle protective housing pivotable toward said needle hub for covering said needle.

2. Needle protection device of claim 1, further comprising:
a sheath (20) for capping said needle prior to its use, said sheath mated to said needle hub after said sleeve has been fitted about said needle hub, said sheath removable from said needle hub after said hub is mated to said luer, said sleeve remaining mated to said luer after the removal of said sheath.

3. Needle protection device of claim 1, wherein said sleeve (2) comprises a proximal portion (4) having a first diameter that enables it to fit over said needle hub and a distal portion (6) having a second diameter larger than said first diameter, an internal circular ledge (16) formed by the intersection of said proximal and distal portions, said distal portion accepting the proximal end of a sheath (20) for capping said needle, the bottom surface of the proximal end of said sheath abutting said ledge when said sheath caps said needle and couples to said hub.

4. Needle protection device of claim 1, wherein said sleeve (2) comprises a groove (8) and wherein said ring (30) is rotatably mounted to said sleeve about said groove.

5. Needle protection device of claim 1, wherein said housing (36) includes at least one internal hook (46) for retaining said needle when said housing is pivoted to cover said needle.

6. Needle protection device of claim 1, wherein said housing (36) comprises one part of a latching mechanism (52) at its proximal end that coacts against an other part of said latching mechanism (56) at either said ring or said sleeve so that when said housing is pivoted to cover said needle, said one and other parts of said latching mechanism interact to prevent said housing from pivoting away from said needle.

7. Needle protection device of claim 1, wherein said container comprises a syringe (64).

8. Needle protection device of claim 1, wherein said housing (36) comprises an elongate one way door (42) that opens when acted against by said needle when said housing is pivoted to cover said needle, but will not open when acted by said needle from inside said housing.

9. Needle protection device of claim 1, wherein said sleeve comprises:
a cylindrical sleeve (2) having a distal portion (6) and a proximal portion (4), the proximal portion having a first diameter dimensioned to fit about the hub (25) of said needle hub and the distal portion having a second diameter dimensioned to allow a sheath (20) to cap a needle (48) extending through said distal portion from said needle hub, said sheath having a proximal opening (18) dimensioned to frictionally couple to said needle hub when said sheath caps said needle, said sleeve including a groove (8) formed about said distal portion, wherein said ring having connected thereto said needle protective housing being rotatably mounted about said groove, said proximal portion of said sleeve adaptable to mate with a luer end of a syringe along with said needle hub, wherein once mated to said syringe said sleeve is fixedly coupled to the luer end of said syringe while said needle protective housing is rotatable about said sleeve.

10. Needle protection device of claim 9, wherein the at least one flange (24) of said sleeve is formed at the proximal portion of said sleeve to enable said sleeve to be threadedly mated to the luer end (66) of said syringe (64) along with said needle hub.

## Patentansprüche

1. Nadelschutzvorrichtung, aufweisend:
eine an einen Nadelansatz (28), von dem sich eine Nadel (48) erstreckt, anbringbare Hülse (2), die mindestens einen Flansch (24) aufweist, der einen sich von dem proximalen Ende des Nadelansatzes erstreckenden Flansch (26) überlagert; und
ein mit einem drehbar an der Hülse befestigten Ring (30) verbundenes Nadelschutzgehäuse (36);
wobei die Hülse über ein Gewinde mit einem Luer (66) eines Flüssigkeit aufnehmenden oder abgebenden Behälters (64) zugleich oder nachdem der Nadelansatz mit dem Luer verbunden wurde derart verbunden wird, dass die Hülse zusammen mit dem Nadelansatz starr mit dem Behälter gekoppelt ist während das Nadelschutzgehäuse rotierbar um die Hülse angeordnet ist, wobei das Nadelschutzgehäuse zum Abdecken der Nadel schwenkbar in Richtung des Nadelansatzes angeordnet ist.

2. Nadelschutzvorrichtung nach Anspruch 1, ferner aufweisend:
eine Umhüllung (20) zum Abdecken der Nadel vor ihrer Verwendung,
wobei die Umhüllung mit dem Nadelansatz verbunden wird nachdem die Hülse an dem Nadelansatz angebracht worden ist, wobei die Umhüllung von dem Nadelansatz entfernbar ist nachdem der Ansatz mit dem Luer verbunden wird und wobei die Hülse nach dem Entfernen der Umhüllung mit dem Luer verbunden bleibt.

3. Nadelschutzvorrichtung nach Anspruch 1, wobei die Hülse (2) einen proximalen Abschnitt (4) mit einem ersten Durchmesser, der es erlaubt, diesen an dem Nadelansatz anzubringen, einen distalen Abschnitt (6) mit einem zweiten Durchmesser, der größer als der erste Durchmesser ist, und eine innenliegende kreisförmige Kante (16), die durch den Übergang zwischen dem proximalen und distalen Abschnitt gebildet wird, aufweist, wobei der distale Abschnitt das proximale Ende einer Umhüllung (20) zum Abdecken der Nadel aufnimmt, wobei die Grundfläche des proximalen Endes der Umhüllung an der Kante anliegt, wenn die Umhüllung die Nadel abdeckt und mit dem Ansatz verbunden ist.

4. Nadelschutzvorrichtung nach Anspruch 1, wobei die Hülse (2) eine Nut (8) umfasst und wobei der Ring (30) rotierbar innerhalb der Nut an der Hülse befestigt ist.

5. Nadelschutzvorrichtung nach Anspruch 1, wobei das Gehäuse (36) mindestens einen innenliegenden Haken (46) zum Halten der Nadel aufweist, wenn das Gehäuse zum Abdecken der Nadel verschwenkt ist.

6. Nadelschutzvorrichtung nach Anspruch 1, wobei das Gehäuse (36) an seinem proximalen Ende einen Teil eines Verriegelungsmechanismus (52) umfasst, der mit einem anderen Teil des Verriegelungsmechanismus (56) entweder an dem Ring oder der Hülse zusammenwirkt, so dass, wenn das Gehäuse zum Abdecken der Nadel verschwenkt wird, dieses und andere Teile des Verriegelungsmechanismus zusammenwirken, um ein Wegschwenken des Gehäuses von der Nadel zu verhindern.

7. Nadelschutzvorrichtung nach Anspruch 1, wobei der Behälter eine Spritze (64) aufweist.

8. Nadelschutzvorrichtung nach Anspruch 1, wobei das Gehäuse (36) eine längliche Einwegtür (42) aufweist, die sich öffnet, wenn die Nadel beim Verschwenken des Gehäuses zum Abdecken der Nadel auf sie einwirkt, die sich aber nicht öffnet, wenn die Nadel von innerhalb des Gehäuses auf sie einwirkt.

9. Nadelschutzvorrichtung nach Anspruch 1, wobei die Hülse aufweist:
eine zylindrische Hülse (2), die einen distalen Abschnitt (6) und einen proximalen Abschnitt (4) aufweist, wobei der proximale Abschnitt einen ersten Durchmesser aufweist, der derart bemessen ist, dass er auf den Ansatz (25) des Nadelansatzes passt und wobei der distale Abschnitt einen zweiten Durchmesser aufweist, der derart bemessen ist, dass es einer Umhüllung (20) möglich ist, eine sich von dem Nadelansatz durch den distalen Abschnitt erstreckende Nadel (48) abzudecken, wobei die Umhüllung eine proximale Öffnung (18) aufweist, die derart bemessen ist, dass diese reibschlüssig mit dem Nadelansatz verbindbar ist, wenn die Umhüllung die Nadel abdeckt,
wobei die Hülse eine an dem distalen Abschnitt ausgebildete Nut (8) aufweist, wobei das Nadelschutzgehäuse bei einem in der Nut angeordneten Ring rotierbar um die Nut befestigt ist, wobei der proximale Abschnitt der Hülse geeignet ist, um mit einem Ende des Luers der Spritze gemeinsam mit dem Nadelansatz verbunden zu werden, wobei die einmal mit der Spritze verbundene Hülse fest mit dem Ende des Luers der Spritze verbunden ist während das Nadelschutzgehäuse rotierbar um die Hülse angeordnet ist.

10. Nadelschutzvorrichtung nach Anspruch 9, wobei der mindestens eine Flansch (24) der Hülse an dem proximalen Abschnitt der Hülse ausgebildet ist, um es der Hülse zu ermöglichen, gemeinsam mit dem Nadelansatz mit dem Ende des Luers (66) der Spritze (64) über ein Gewinde verbunden zu werden.

## Revendications

1. Dispositif de protection d'aiguille, comprenant:
un manchon (2) pouvant être adapté autour d'un porte-aiguille (28) à partir duquel une aiguille (48) s'étend, ledit manchon comportant au moins une bride (24) qui surplombe une bride (26) qui s'étend à partir de l'extrémité proximale dudit porte-aiguille; et
un logement de protection d'aiguille (36) qui est attaché à un anneau (30) monté, de façon rotative, autour dudit manchon;
dans lequel ledit manchon est agencé par vissage sur un embout Luer (66) d'un récipient (64) de collecte ou de distribution de fluide en même temps que ledit porte-aiguille est agencé sur ledit embout Luer ou après que ledit porte-aiguille ait été agencé sur ledit embout Luer, de telle sorte que ledit manchon soit couplé fixement audit récipient avec ledit porte-aiguille alors que ledit logement de protection d'aiguille peut tourner par rapport audit manchon, ledit logement de protection d'aiguille pouvant pivoter en direction dudit porte-aiguille afin de couvrir ladite aiguille.

2. Dispositif de protection d'aiguille selon la revendication 1, comprenant en outre:
une gaine (20) pour envelopper ladite aiguille avant son utilisation, ladite gaine étant couplée audit porte-aiguille après que ledit manchon ait été agencé autour dudit porte-aiguille, ladite gaine pouvant être enlevée dudit porte-aiguille une fois que ledit porte-aiguille est couplé audit embout Luer, ledit manchon restant couplé audit embout Luer après l'enlèvement de ladite gaine.

3. Dispositif de protection d'aiguille selon la revendication 1, dans lequel ledit manchon (2) comprend une partie proximale (4) qui présente un premier diamètre qui lui permet d'être agencé sur ledit porte-aiguille, et une partie distale (6) qui présente un deuxième diamètre, qui est supérieur audit premier diamètre, un rebord circulaire interne (16) formé par l'intersection desdites parties proximale et distale, ladite partie distale acceptant l'extrémité proximale d'une gaine (20) pour envelopper ladite aiguille, la surface inférieure de l'extrémité proximale de ladite gaine butant contre ledit rebord lorsque ladite gaine enveloppe ladite aiguille et est couplée audit porte-aiguille.

4. Dispositif de protection d'aiguille selon la revendication 1, dans lequel ledit manchon (2) comporte une rainure (8), et dans lequel ledit anneau (30) est monté de façon rotative sur ledit manchon autour de ladite rainure.

5. Dispositif de protection d'aiguille selon la revendication 1, dans lequel ledit logement (36) comprend au moins un crochet interne (46) destiné à retenir ladite aiguille lorsque ledit logement pivote pour couvrir ladite aiguille.

6. Dispositif de protection d'aiguille selon la revendication 1, dans lequel ledit logement (36) comprend une partie d'un mécanisme de verrouillage (52) à son extrémité proximale qui co-agit contre une autre partie dudit mécanisme de verrouillage (56) soit audit anneau, soit audit manchon, de telle sorte que lorsque ledit logement pivote pour couvrir ladite aiguille, lesdites première et deuxième parties dudit mécanisme de verrouillage interagissent pour empêcher ledit logement de pivoter à l'écart de ladite aiguille.

7. Dispositif de protection d'aiguille selon la revendication 1, dans lequel ledit récipient comprend une seringue (64).

8. Dispositif de protection d'aiguille selon la revendication 1, dans lequel ledit logement (36) comprend une porte à une voie allongée (42) qui s'ouvre lorsqu'elle est poussée par ladite aiguille lorsque ledit logement pivote pour couvrir ladite aiguille, mais qui ne s'ouvrira pas lorsqu'elle sera poussée par ladite aiguille depuis l'intérieur dudit logement.

9. Dispositif de protection d'aiguille selon la revendication 1, dans lequel ledit manchon comprend:
un manchon cylindrique (2) comprenant une partie distale (6) et une partie proximale (4), la partie proximale présentant un premier diamètre dimensionné de manière à s'adapter autour du moyeu (25) dudit porte-aiguille, et la partie distale présentant un deuxième diamètre dimensionné de manière à permettre à une gaine (20) d'envelopper une aiguille (48) qui s'étend à travers ladite partie distale à partir dudit porte-aiguille, ladite gaine comportant une ouverture proximale (18) dimensionnée pour se coupler par frottement audit porte-aiguille lorsque ladite gaine enveloppe ladite aiguille, ledit manchon comportant une rainure (8) formée autour de ladite partie distale, dans lequel ledit anneau présente, connecté à celui-ci, ledit logement de protection d'aiguille qui est monté de façon rotative autour de ladite rainure, ladite partie proximale dudit manchon étant adaptable pour être couplée à un embout Luer d'une seringue avec ledit porte-aiguille, dans lequel une fois couplé à ladite seringue, ledit manchon est couplé fixement à l'embout Luer de ladite seringue alors que ledit logement de protection d'aiguille peut tourner autour dudit manchon.

10. Dispositif de protection d'aiguille selon la revendication 9, dans lequel ladite au moins une bride (24) dudit manchon est formée à la partie proximale dudit manchon de manière à permettre audit manchon d'être couplé par vissage à l'embout Luer (66) de ladite seringue (64) avec ledit porte-aiguille.
